Europäisches Patentamt

European Patent Office

Office européen des brevets

(19)

(11) Veröffentlichungsnummer : **0 121 543**
**B1**

## (12) EUROPÄISCHE PATENTSCHRIFT

(45) Veröffentlichungstag der Patentschrift :
28.05.86

(21) Anmeldenummer : 83903196.0

(22) Anmeldetag : 07.10.83

(86) Internationale Anmeldenummer :
PCT/HU 83/00052

(87) Internationale Veröffentlichungsnummer :
WO/8401502 (26.04.84 Gazette 84/11)

(51) Int. Cl.⁴ : **A 61 K 31/12, A 61 K 31/19,
A 61 K 33/22, A 61 K 9/20**

(54) KONTRAZEPTIVE SCHEIDENTABLETTEN UND VERFAHREN ZU DEREN HERSTELLUNG.

(30) Priorität : 07.10.82 HU 321182

(43) Veröffentlichungstag der Anmeldung :
17.10.84 Patentblatt 84/42

(45) Bekanntmachung des Hinweises auf die Patenterteilung : 28.05.86 Patentblatt 86/22

(84) Benannte Vertragsstaaten :
AT BE CH DE FR GB LI NL SE

(56) Entgegenhaltungen :
GB-A- 966 685
US-A- 3 062 715
US-A- 4 068 001

(73) Patentinhaber : KOVACS, András
Bajcsy Zs. ut 19/a
H-1065 Budapest (HU)

SZEBENI, Rudolf
Béke u. 48
H-2131 Alsogöd (HU)

KÖSZEGI, Béla
Corvin Körut 52
H-1192 Budapest (HU)

(72) Erfinder : KOVACS, András
Bajcsy Zs. ut 19/a
H-1065 Budapest (HU)
Erfinder : SZEBENI, Rudolf
Béke u. 48
H-2131 Alsogöd (HU)
Erfinder : KÖSZEGI, Béla
Corvin Körut 52
H-1192 Budapest (HU)

(74) Vertreter : Sandmair, Kurt, Dr. et al
Patentanwälte Schwabe, Sandmair, Marx Stuntzstrasse 16
D-8000 München 80 (DE)

**Beschreibung**

Gebiet der Erfindung

Die Erfindung betrifft hormonfreie kontrazeptive Scheidentabletten, sowie ein Verfahren zu deren Herstellung.

Stand der Technik

Es ist bekannt, dass zur Verhinderung des Eintritts der unerwünschten Schwangerschaft — ausser der Operationen — im wesentlichen drei Verfahren bekannt sind. Diese sind der Gummikondom, die intra-uterinen Mittel und die Scheidenpessar (mechanische Mittel), die durch die Anwendung von hormonalen Mitteln und von hormonfreien lokalen spermiciden Mitteln das Zustandekommen der Schwangerschaft verhindern.

Alle bekannten Lösungen haben Nachteile, die eine allgemeine Anwendung nicht ermöglichen. Es ist bekannt, dass nicht alle konzeptionsfähigen Alterklassen die hormonalen Mittel nützen können. Gewisse Personen, obwohl sie wegen ihres Alters diese Mittel einnehmen könnten, kommen wegen der Nebenwirkungen um deren Anwendung. Unter den mechanischen Mitteln rufen der Gummikondom und das Scheidenpessar bei gewissen Personen ungemütliches Gefühl hervor bzw. beanspruchen diese illusionzersetzende Vorbereitungen.

Obwohl diese Nachteile bei den intrauterinen Mitteln nicht vorkommen, kann nicht jedermann diese tragen, anderseits darf die jüngste Alterklasse der konzeptions-fähigen Frauen diese Mittel nicht nutzen. So kann es nur einen adventiven Nachteil bedeuten; dass diese Mittel nur durch den Arzt aufgesetzt werden können. Bei den hormonfreien Mitteln gibt es keine alterische Gegenanzeige, diese werden aber selten in sich angewandt werden, da ihre Zuverlässigkeit nicht ausreichend ist.

Die Wirksamkeit der bekannten kontrazeptiven Mittel und Methoden kann durch die sog. Pearl-Index charakterisiert werden. Diese Zahl zeigt es, dass unter 100 Frauen, die die bestimmte Empfängnisverhütung anwenden, wieviele während eines Jahres schwanger sein werden. Der Pearl-Index der kontrazeptiven Mittel und Methode ist in der nachfolgenden Tabelle zusammengefasst :

| | |
|---|---|
| Scheidenspülung | 29,3-40,8 |
| unterbrochener Begattungsakt | 12-38 |
| Ogino-Knaus-Regel | 12-34,5 |
| Schaumtablette | 11,9-42,8 |
| Scheidenglobulus | 7,7-42,3 |
| Scheidewand | 6,1-33,6 |
| Gelee | 6,4-41 |
| Scheidenpessar | 6,0-29 |
| Gummikondom | 6-28 |
| intrauterine Mittel | 0,9-8 |
| hormonale Mittel | 0-1,7 |

Aus den obigen Angaben ist es gut ersichtlich, dass nur die hormonalen Mittel eine ausreichende Sicherheit geben, die Wirksamkeit der nur chemische Wirkstoffe enthaltenden Mittel bleibt weit unter der Wirksamkeit der hormonalen Mittel.

Das Wesen der Erfindung

Ziel unserer Erfindung ist eine hormonfreie kontrazeptive Scheidentablette auszuarbeiten, die die Wirksamkeit der hormonalen Mittel aufweist, doch verfügt nicht über deren schädliche Nebenwirkungen und kann unabhängig von dem Alter und dem Gesundheitszustand angewandt werden.

Das Vitamin $K_3$ und dessen Natriumbisulfit-Addukt werden in der Therapie verbreitet angewandt, z. B. zur Behandlung von Okklusionsikterus, Operationsvor- und Nachbehandlung bei Cholämie, Gallenfistel, Kolitis ulcerosa, Dysenterie, Steatorrhoe, Sprue, Zöliakie, Neugeborenenhämophilie, Gelbsucht, Salizylismus, Purpura, Thrombopenia, Serumkrankheit, Urtikaria, Hämoptoe. Die kontrazeptive Wirkung der erwähnten Substanzen ist aber in der Literatur nicht beschrieben.

Die therapeutische Anwendung der Borsäure und der Weinsäure ist auch bekannt. Die Borsäure wird wegen ihrer schwachen dezinfizierenden Wirkung zur Spülung der Körperhöhlen, die Weinsäure wegen ihrer schwachen sauren Reaktion zur Einstellung des pH-Wertes der Mittel angewandt.

Es wurde unerwartet gefunden, dass eine dem obigen Ziel entsprechende kontrazeptive Scheidentablette erhalten wird, wenn man

0,2 bis 0,3 Gewichtsteil Borsäure,
10,0 bis 20 Gewichtsteile Weinsäure,

1 bis 2 Gewichsteile Vitamin $K_3$-Natriumbisulfit-Addukt,
0,8 bis 1,2 Gewichtsteile Polyvinylpyrrolidon,
2 bis 5 Gewichtsteile Magnesiumstearat,
8 bis 12 Gewichtsteile Carboxymethylcellulose,
8 bis 12 Gewichtsteile Laktose,
50 bis 65 Gewichtsteile mikrokristalline Cellulose

homogenisiert, danach zu Tabletten presst.

Vorteilhaft werden Tabletten hergestellt, die 5 mg an Vitamin $K_3$-Natriumbisulfit-Addukt pro Tablette enthalten. Diese Tablette hat ein Gesamtgewicht von 500 mg.

Die erfindungsgemässe Scheidentablette soll vor der Anwendung mit Wasser angefeuchtet, danach in das hintere Scheidengewölbe gesetzt werden. Die Tabletten zerfallen hier infolge der Nässe schnell und bilden eine Suspension, die den Zervix einzieht und die daran anwesenden Spermien lähmt. Die Rückstände der Mittel können durch Scheidenspülung leicht entfernt werden. Die Anwendung der Tabletten verursacht kein ungemütliches Gefühl, die in der Scheide zerfallenen Tabletten üben keine schädlichen Nebenwirkungen aus.

Bevorzugte Verwirklichung der Erfindung

Weitere Einzelheiten der Erfindung werden ohne irgendeine Einschränkung durch die folgenden Ausführungsbeispiele gezeigt.

Beispiel 1

Aus 2,0 g pulverisierter Borsäure, 100,0 g pulversierter Weinsäure und 100,0 g kristalliger Laktose wird ein homogenisiertes Pulvergemisch hergestellt. Gesondert werden auch 10,0 g Vitamin $K_3$-Natriumbisulfit-Addukt, 10,0 g Polyvinylpyrrolidon (Polyplasdon XL), 30,0 g Magnesiumstearat und 100,0 g Carboxymethylcellulose homogenisiert. Die zwei Pulvergemische werden versetzt und 648,0 g mikrokristalline Cellulose wird zugegeben. Aus dem erhaltenen 1 000,0 g Pulvergemisch werden 2 000 Tabletten mit einem Gewicht von 500 mg und einem Durchmesser von 12 mm ohne einem Rand gepresst.

Einige Charakteristiken der erhaltenen Tabletten sind hier aufgeführt :
Verschleissfestigkeit (ERWEKA-TAP) :

| nach 5 Minuten | nach 10 Minuten |
|---|---|
| 1,46 % | 3,02 % |
| 1,29 % | 2,88 % |

Druckfestigkeit (ERWEKA) : 14,3 N
Durchschnittshöhe der Tabletten : 4,52 mm
Zerfallzeit (Kolbenmethode nach PA.Mg.VI) : 9-10 Sekunden.

Beispiel 2

Tabletten werden mit der folgenden Zusammensetzung nach dem Verfahren des Beispiels 1 hergestellt :

| | |
|---|---|
| Borsäure | 5,0 mg |
| Weinsäure | 50,0 mg |
| Vitamin $K_3$-Natriumbisulfit-Addukt | 5,0 mg |
| Polyvinylpyrrolidon | 5,0 mg |
| Magnesiumstearat | 15,0 mg |
| Carboxymethylcellulose | 50,0 mg |
| Laktose | 50,0 mg |
| mikrokristalline Cellulose | 320,0 mg |
| | 500,0 mg |

Beispiel 3

Tabletten werden mit der folgenden Zusammensetzung nach dem Verfahren des Beispiels 1 hergestellt :

| | |
|---|---|
| Borsäure | 15,0 mg |
| Weinsäure | 50,0 mg |

| | |
|---|---|
| Vitamin K$_3$-Natriumbisulfit-Addukt | 5,0 mg |
| Polyvinylpyrrolidon | 5,0 mg |
| Magnesiumstearat | 15,0 mg |
| Carboxymethylcellulose | 50,0 mg |
| Laktose | 50,0 mg |
| mikrokristalline Cellulose | 310,0 mg |

500,0 mg

## Beispiel 4

Tabletten werden mit der folgenden Zusammensetzung nach dem Verfahren des Beispiels 1 hergestellt :

| | |
|---|---|
| Borsäure | 1,0 mg |
| Weinsäure | 100,0 mg |
| Vitamin K$_3$-Natriumbisulfit-Addukt | 5,0 mg |
| Polyvinylpyrrolidon | 5,0 mg |
| Magnesiumstearat | 15,0 mg |
| Carboxymethylcellulose | 50,0 mg |
| Laktose | 50,0 mg |
| mikrokristalline Cellulose | 274,0 mg |

500,0 mg

## Beispiel 5

Tabletten werden mit der folgenden Zusammensetzung hergestellt :

| | |
|---|---|
| Borsäure | 1,0 mg |
| Weinsäure | 50,0 mg |
| Vitamin K$_3$-Natriumbisulfit-Addukt | 10,0 mg |
| Polyvinylpyrrolidon | 5,0 mg |
| Magnesiumstearat | 15,0 mg |
| Carboxymethylcellulose | 50,0 mg |
| Laktose | 50,0 mg |
| mikrokristalline Cellulose | 319,0 mg |

500,0 mg

## Beispiel 6

Tabletten werden mit der folgenden Zusammensetzung hergestellt :

| | |
|---|---|
| Borsäure | 5,0 mg |
| Weinsäure | 50,0 mg |
| Vitamin K$_3$-Natriumbisulfit-Addukt | 10,0 mg |
| Polyvinylpyrrolidon | 5,0 mg |
| Magnesiumstearat | 15,0 mg |
| Carboxymethylcellulose | 50,0 mg |
| Laktose | 50,0 mg |
| mikrokristalline Cellulose | 315,0 mg |

500,0 mg

## Beispiel 7

Tabletten werden mit der folgenden Zusammensetzung hergestellt :

| | |
|---|---|
| Borsäure | 15,0 mg |
| Weinsäure | 50,0 mg |
| Vitamin K$_3$-Natriumbisulfit-Addukt | 10,0 mg |
| Polyvinylpyrrolidon | 5,0 mg |
| Magnesiumstearat | 15,0 mg |

| Carboxymethylcellulose | 50,0 mg |
| Laktose | 50,0 mg |
| mikrokristalline Cellulose | 305,0 mg |
| | 500,0 mg |

Beispiel 8

Tabletten werden mit der folgenden Zusammensetzung hergestellt :

| Borsäure | 1,0 mg |
| Weinsäure | 100,0 mg |
| Vitamin $K_3$-Natriumbisulfit-Addukt | 10,0 mg |
| Polyvinylpyrrolidon | 5,0 mg |
| Magnesiumstearat | 15,0 mg |
| Laktose | 50,0 mg |
| Carboxymethylcellulose | 50,0 mg |
| mikrokristalline Cellulose | 269,0 mg |
| | 500,0 mg |

Die erfindungsgemäss hergestellen Tabletten wurden bei human Subjekten in vitro und in vivo untersucht.

In vitro Untersuchung

Es wurden Spermien, die von 32 normalen, normozoospermien Personen gewonnen wurden, untersucht. Aus dem Sperma wurde 1 ml, welches 10 bis 100 Millionen Spermien enthielte, mit 1 ml Suspension, die aus einer Tablette hergestellt wurde, bei + 38 °C auf einem Uhrglas versetzt. Die Bewegung der Spermien wurde beobachtet. Die partielle Immobilisierung erfolgte eine Minute nach der Versetzung und wurde abhängig der Vitalität der Spermien in 5 bis 10 Minuten vollständig geworden. Die immobil gewordenen Spermien wurden Eosinbindner geworden, was den Zellentod bedeutet. Dieser Vorgang ist irreversibel. Zur Wiederbelebung der Spermien waren die Aenderung des pH-Wertes, die Zugabe von frischem Serum und auch andere Methode nicht erfolgreich.

In vivo Modelluntersuchung

Bei Frauen, die im Zeitraum des Eiaustritts waren, wurde die folgende Untersuchung durchgeführt. Die erfindungsgemäss hergestellten Tabletten wurden mit Leitungswasser angefeuchtet, danach in das hintere Scheidengewölbe gesetzt. Nach 5 Minuten wurde die Scheide eröffnet und wurden je 2 ml Sperma, welches 160 bis 240 Millionen Spermien/ml enthielte, auf den Zervix der Frauen gespritzt. Das Instrument der Eröffnung wurde entfernt. Nach 5 und 10 Minuten wurden wieder Eröffnungen durchgeführt und so Muster aus dem Scheideninhalt genommen.

Durch mikroskopische Untersuchung konnte es festgestellt werden, dass die Immobilisierung nach 5 Minuten 30 %, nach 10 Minuten 100 % beträgt.

In vivo Anwendungsversuche

Die Tabletten wurden bei Frauen, bei denen die Konzeptionsfähigkeit bewiesen wurde und die sich bis der Zeit der Untersuchung nur mit dem Ogino-Knaus-Regel geschützt hatten, angewandt. Während eines Jahres erfolgte bei diesen Frauen nur in zwei Fällen die Schwangerschaft. Dies zeigte, dass die Wirksamkeit der erfindungsgemässen Tabletten gleich, wie die der intrauterinen Mittel ist (der Pearl-Index beträgt 2).

Die untersuchten Frauen wurden in je 2-3 Monaten gynäkologischer Untersuchung unterworfen, damit die eventuellen Nebenwirkungen an der Scheide, sowie die schleimhautschädigende Wirkungen bestimmen werden könnten. Solche Fälle kamen unter den geprüften Frauen nicht vor. Aufgrund der obigen kann es festgestellt werden, dass die erfindungsgemässen Scheidentabletten in jeder Hinsicht zur zeitnahen Empfängnisverhütung geeignet sind.

**Patentansprüche**

1. Kontrazeptive Scheidentablette, dadurch gekennzeichnet, dass sie 0,2 bis 3 Gewichtsteile Borsäure, 10 bis 20 Gewichtsteile Weinsäure, 1 bis 2 Gewichtsteile Vitamin $K_3$-Natriumbisulfit-Addukt, 0,8 bis 1,2 Gewichtsteile Polyvinylpyrrolidon, 2 bis 5 Gewichtsteile Magnesiumstearat, 8 bis 12 Gewichtsteile Carboxymethylcellulose, 50 bis 65 Gewichtsteile mikrokristalline Cellulose enthält.

2. Verfahren zur Herstellung von kontrazeptiven Scheidentabletten, dadurch gekennzeichnet, dass man 0,2 bis 3 Gewichtsteile Borsäure, 10 bis 20 Gewichtsteile Weinsäure, 1 bis 2 Gewichtsteile Vitamin $K_3$-Natriumbisulfit-Addukt, 0,8 bis 1,2 Gewichtsteile Polyvinylpyrrolidon, 2 bis 5 Gewichtsteile Magnesiumstearat, 8 bis 12 Gewichtsteile Carboxymethylcellulose, 50 bis 65 Gewichtsteile mikrokristalline Cellulose homogenisiert, danach zu Tabletten presst.

## Claims

1. Contraceptive vaginal tablets, comprising 0.2 to 3 % by weight boric acid, 10 to 20 % by weight tartaric acid, 1 to 2 % by weight vitamine $K_3$-sodium bisulfite-adduct 0.8 to 1.2 % by weight polyvinylpyrrolidone, 2 to 5 % by weight magnesium stearate, 8 to 12 % by weight carboxymethylcellulose, 50 to 65 % by weight microcristalline cellulose.

2. A method for manufacturing contraceptive vaginal tablets, comprising the homogenization of 0.2 to 3 % by weight boric acid, 10 to 20 % by weight tartaric acid, 1 to 2 % by weight vitamine $K_3$-sodium bisulfite-adduct 0.8 to 1.2 % by weight polyvinylpyrrolidone, 2 to 5 % by weight magnesium stearate, 8 to 12 % by weight carboxymethylcellulose 50 to 65 % by weight microcristalline cellulose and compressing the mixture to form tablets.

## Revendications

1. Tablette vaginale contraceptive, caractérisée en ce qu'elle contient 0,2 à 3 parties en poids d'acide borique, 10 à 20 parties en poids d'acide tartrique, 1 à 2 parties en poids de produit d'addition vitamine $K_3$-bisulfite de sodium, 0,8 à 1,2 parties en poids de polyvinylpyrrolidone, 2 à 5 parties en poids de stéarate de magnésium, 8 à 12 parties en poids de carboxyméthyl-cellulose, 50 à 65 parties en poids de cellulose microcristalline.

2. Procédé de fabrication de tablettes vaginales contraceptives, caractérisé en ce qu'on homogénéise 0,2 à 3 parties en poids d'acide borique, 10 à 20 parties en poids d'acide tartrique, 1 à 2 parties en poids de produit d'addition vitamine $K_3$-bisulfite de sodium, 0,8 à 1,2 partie en poids de polyvinylpyrrolidone, 2 à 5 parties en poids de stéarate de magnésium, 8 à 12 parties en poids de carboxyméthyl-cellulose, 50 à 65 parties en poids de cellulose microcristalline, et en ce que par la suite on les comprime en tablettes.